# EUROPEAN PATENT SPECIFICATION

(11) **EP 1 274 386 B1**
(45) Date of publication and mention of the grant of the patent: **02.02.2005**
(21) Application number: 01920032.8
(22) Date of filing: 26.03.2001
(51) Int. Cl.: A61F 13/64

(54) **ABSORBENT ARTICLE PROVIDED WITH A BELT**
ABSORBIERENDER ARTIKEL MIT EINEM GÜRTEL
ARTICLE ABSORBANT DOTE D'UNE CEINTURE

(30) Priority: 31.03.2000 SE 0001174
(43) Date of publication of application: 15.01.2003
(73) Proprietor: SCA Hygiene Products AB, 405 03 Göteborg (SE)
(72) Inventor: KARLSSON, Katharina, S-438 94 Härryda (SE); HERMANSSON, Kent, S-426 54 Västra Frölunda (SE)
(74) Representative: Egeröd, Lisbeth
(86) International application number: PCT/SE2001/000647
(87) International publication number: WO 2001/074283

(56) References cited:
- EP-A1- 0 463 276
- WO-A1-86/04812
- WO-A1-94/26222
- US-A- 4 317 449

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion having side edges and a crotch portion therebetween, and further is provided with a pair of belt portions attached to the rear portion of the article and which are intended to be fastened together around the waist of the wearer and where said front portion is provided with attachment means intended to be attached to the belt portions, in such a way that the article will assume a pantlike shape, where the belt portions form a part of the waist portions of the absorbent article.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e g EP-A-0 287 388, EP-A-0 409 307, EP-A-0 605 012 and FR-A-2 586 558, to attach the front and rear portions of the article by means of a belt, at which the possibilities to adjust the fit are improved. The belt further provides a simplified change of diaper or incontinence guard. The ends of the belt portions can however be difficult to grasp when the article shall be applied to a wearer, especially if the wearer is lying down and the belt ends get caught under the wearer.

Through WO 86/04812 it is previously known to fold the attachment straps of a diaper, i e the straps that carry the attachment means that fasten up the front and rear portions of the diaper, in accordion-like fashion before they are applied to the diaper in order to simplify manufacture and packaging of the products. The folded attachment straps are kept together by a weak adhesive applied in narrow zones in order to be easily released and unfolded when the diaper is ready for use. The attachment straps do however not constitute a belt as defined in the present invention.

Through EP-A-0 463 276 it is previously known to keep extensible fastener tabs in pockets at each side edge at the rear portion of the diaper. The fastener tabs are elastic and can when used be extended and stretched from the pockets. Neither in this case the fastener tabs constitute a belt which is to be attached around the waist of the user.

Document WO-A-94/26222 shows the features of the preamble of claim 1.

### Object and most important features of the invention

The object of the present invention is to accomplish a belt-provided absorbent article, such as a diaper or incontinence guard, in which the application of the product on the user is simplified by the fact that the belt portions during application are easily accessible even if the user is lying down. This has according to the invention been solved by the fact that the belt portions before use are folded in accordion-like fashion and each form an accordion-like folded package which is arranged in a pocket at each side edge of the rear portion of the absorbent article, and can be extended from said pocket and unfolded when the article is to be used, after which the belt portions may be fastened together around the waist of the wearer.

According to a preferred embodiment said pocket is formed between the liquid pervious topsheet and the liquid impervious backsheet of the rear portion.

Preferably the accordion folds of the belt portions are kept together by breakable seals.

### Description of drawings

The invention will in the following be closer described with reference to an embodiment shown in the accompanying drawings.

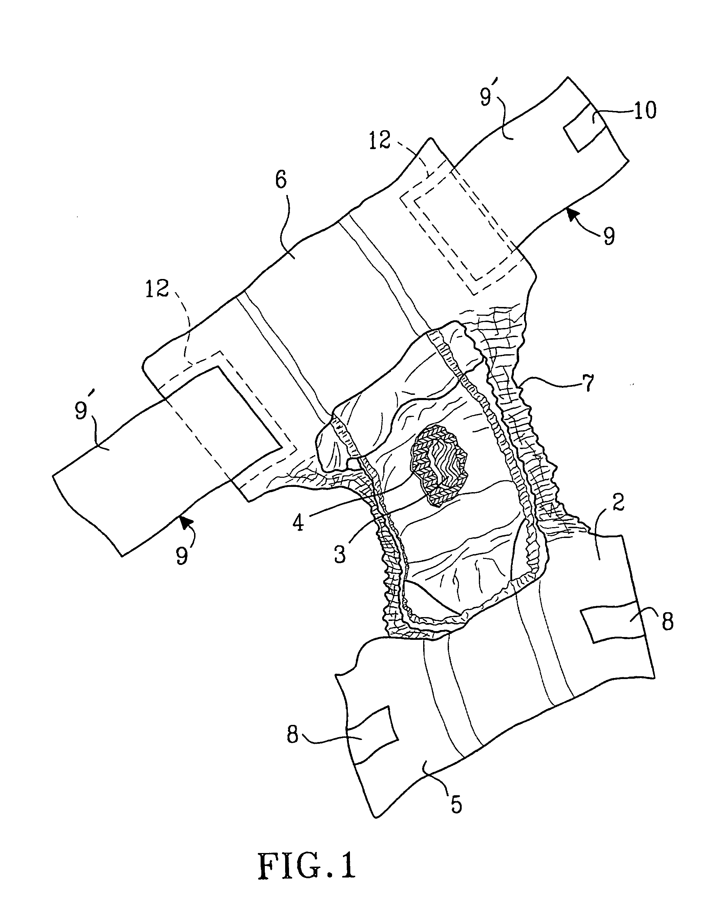

Fig. 1 shows schematically a perspective view of a diaper or incontinence guard according to the invention having the belt portions folded and placed in pockets at the rear portion.

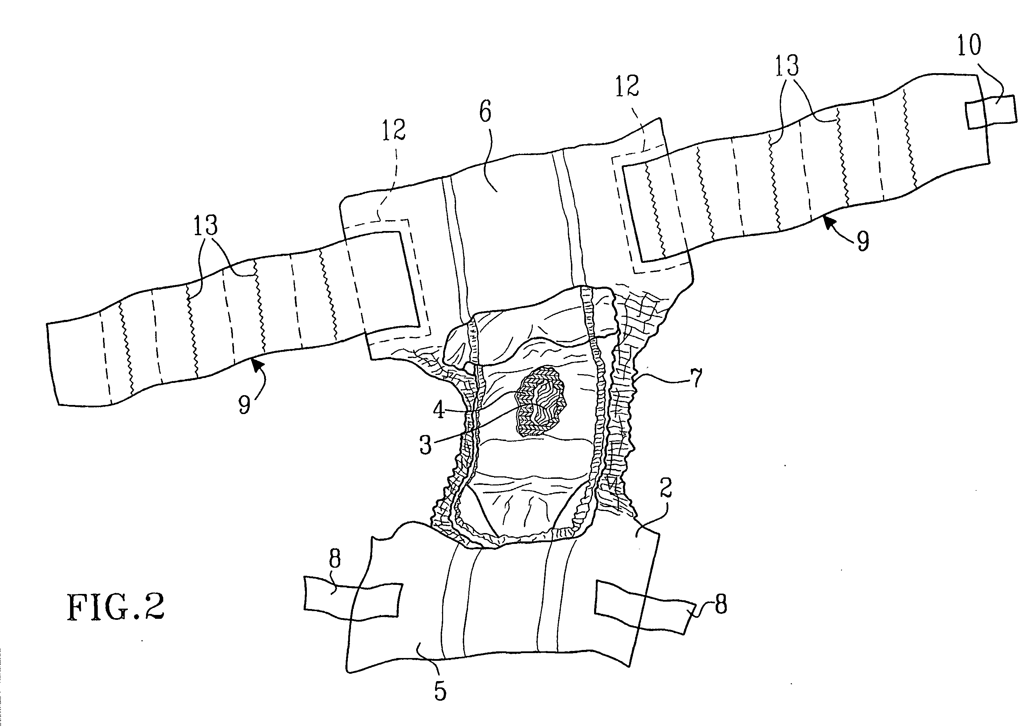

Fig. 2 shows the diaper according to Fig. 1 but having the belt portions extended and unfolded.

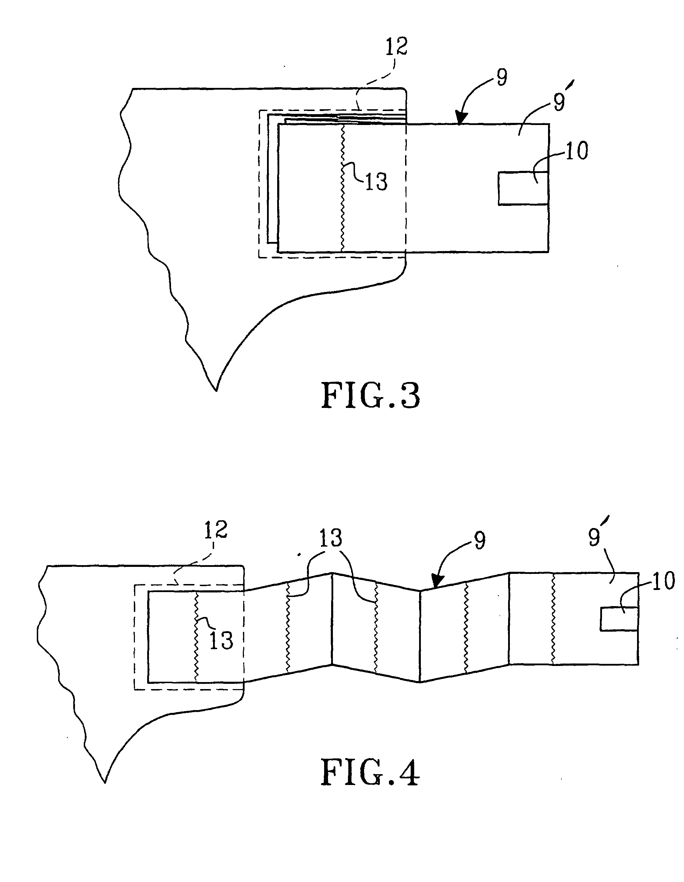

Fig. 3 and 4 show on a larger scale one of the belt portions in folded and unfolded positions respectively.

### Description of an embodiment

The drawing shows an embodiment of a diaper or incontinence guard 1 comprising a liquid permeable topsheet 2, a liquid impermeable backsheet 3 and an absorbent body 4 enclosed therebetween. The liquid permeable topsheet 2 can consist of a nonwoven material, e g a spunbond material of continuous filaments, a meltblown material or a bonded carded fibrous web. The liquid impermeable backsheet 3 may consist of a plastic film, a nonwoven material coated with a liquid impervious material or a hydrophobic nonwoven material which resists liquid penetration.

The topsheet 2 and the backsheet material 3 has a somewhat greater extension in the plane than the absorbent body 4 and extends outside the edges thereof. The layers 2 and 3 are connected to each other within the projecting portions thereof, e g by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper/incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of adhesive tape portions 8 or other type of attachment means such as hooks and loops fasteners of the touch-and-close type.

A pair of belt portions 9 are with one end attached, e g glued or ultrasonically welded, to the rear portion 6 of the diaper. The belt portions 9 are with their opposite ends intended to be fastened together, e g by means of tape tab 10 which is taped against the outside of the opposite belt portion. Instead of a tape tab there may be another optional attachment means, such as hook-and-loop type fasteners. The attachment tapes 8 of the front portion 5 or corresponding attachment means are intended to be attached against the outsides of the belt portions 9 in order to fasten together the diaper/incontinence guard to the desired pantlike shape.

The width of the belt portions 9 should be between 5-20 cm, preferably between 7-15 cm.

The belt portions 9 are preferably a laminate of a carrier material, which forms the outside of the belt, and a soft nonwoven, which forms the inside of the belt intended to be in direct contact with the skin of the user.

A suitable nonwoven material can be a spunbond material of e g polypropylene- or polyethylene fibres. Conjugate fibres may also be used. Another suitable nonwoven material can be a carded thermobonded material of e g polypropylene- , polyester- or conjugate fibres.

As a carrier material there can be used a plastic film or another appropriate material, e g a nonwoven. The carrier material should be adapted to function as a reception surface for the attachment means 8 and 10, at which in those cases the attachment means are tape tabs, a plastic film is suitable. In case other types of attachment means are used instead of tape tabs, e g hook-and-loop type fasteners, another type of carrier material is suitable which may function as a reception surface for the attachment means in question. Also elastic laminates are suitable to use as material in the belt portions.

The belt portions 9 are before use folded in accordion-like fashion and each form an accordion-like folded package 11 arranged in pocket 12 at each side edge of the rear portion 6 of the diaper/incontinence guard. A flap 9' of each belt portion 9 projects preferably outside the pocket 12, so that it may easily be grasped. This pocket 12 preferably constitutes a space between the topsheet 2 and the backsheet material 3 where these are not joined together. The pocket may also constitute a separate part attached on the outside of the backsheet material 3 alternatively on the inside of the topsheet material 2.

The accordion folds 9 of the belt portions are kept together by means of easily breakable seals 13, e g glue strings/glue spots or weld seams/weld spots provided by ultrasonic or heat. When the diaper/incontinence guard is to be applied on the user the belt portions 9 are easily accessible for the nursing staff and by a light jerk in the flap 9' projecting from pocket 12 the seal is broken and the belt portions 9 can be unfolded and attached around the waist of the user. The tape tabs 8 of the front portion 5 or corresponding attachment means can then be attached against the outside of the belt portions 9 in order to fasten together the diaper/incontinence guard to the desired pantlike shape.

## Claims

1. Absorbent article such as a diaper and an incontinence guard comprising a liquid permeable topsheet (2), a liquid impermeable backsheet (3) and an absorbent body (4) enclosed therebetween, said article having a front portion (5), a rear portion (6) having side edges and a crotch portion (7) therebetween, and further is provided with a pair of belt portions (9) attached to the rear portion (6) of the article and which are intended to be fastened together around the waist of the wearer and where said front portion (5) is provided with attachment means (8) intended to be attached to the belt portions (9), in such a way that the article will assume a pantlike shape, where the belt portions (9) form a part of the waist portions of the absorbent article
**characterized in**
**that** the belt portions (9) before use are folded in accordion-like fashion and each forms an accordion-like folded package (11) which is arranged in a pocket (12) at each side edge of the rear portion (6) of the absorbent article, and can be extended from said pocket and unfolded when the article is to be used, after which the belt portions may be fastened together around the waist of the wearer.

2. Absorbent article as claimed in claim 1,
**characterized in**
**that** said pocket (12) is formed between the liquid pervious topsheet (2) and the liquid impervious backsheet (3) of the rear portion (3).

3. Absorbent article as claimed in claim 1 or 2,
**characterized in**
**that** the accordion folds (9') of the belt portions (9) are kept together by breakable seals (13).

## Patentansprüche

1. Absorbierender Gegenstand, wie beispielsweise eine Windel und ein Inkontinenzschutz, umfassend eine flüssigkeitsdurchlässige Oberlage (2), eine flüssigkeitsundurchlässige Decklage (3) und einen Absorptionskörper (4), der dazwischen eingeschlossen ist, wobei der Gegenstand einen Vorderabschnitt (5), einen Hinterabschnitt (6) mit Seitenkanten und einen Schrittabschnitt (7) dazwischen aufweist, und ferner mit einem Paar Gürtelabschnitte (9), die an dem Hinterabschnitt (6) des Gegenstands angebracht und dazu gedacht sind, zusammen um die Taille des Trägers befestigt zu werden, und wobei der Vorderabschnitt (5) mit einem Befestigungsmittel (8) versehen ist, das dazu gedacht ist, an den Gürtelabschnitten (9) angebracht zu werden, so dass der Gegenstand eine hosenähnliche Form annehmen wird, wobei die Gürtelabschnitte (9) einen Teil der Taillenabschnitte des absorbierenden Gegenstand bilden,
**dadurch gekennzeichnet,**
**dass** die Gürtelabschnitte (9) vor der Verwendung in einer Ziehharmonika-ähnlichen Art gefaltet sind und jeweils eine Ziehharmonika-ähnlich gefaltete Ansammlung (11) bilden, die in einer Tasche (12) an jeder Seitenkante des Hinterabschnitts (6) des absorbierenden Gegenstands angeordnet ist, und aus der Tasche gezogen und entfaltet werden können, wenn der Gegenstand zu verwenden ist, wonach die Gürtelabschnitte um die Taille des Trägers aneinander befestigt werden können.

2. Absorbierender Gegenstand nach Anspruch 1,
**dadurch gekennzeichnet,**
**dass** die Tasche (12) zwischen der flüssigkeitsdurchlässigen Oberlage (2) und der flüssigkeitsundurchlässigen Decklage (3) des Hinterabschnitts (6) ausgebildet ist.

3. Absorbierender Gegenstand nach Anspruch 1 oder 2,
**dadurch gekennzeichnet,**
**dass** die Ziehharmonika-Falten (9') der Gürtelabschnitte (9) durch aufbrechbare Siegelungen (13) zusammengehalten werden.

## Revendications

1. Article absorbant tel qu'une couche-culotte et une protection contre l'incontinence comprenant une feuille supérieure (2) perméable aux liquides, une feuille arrière (3) imperméable aux liquides et un corps absorbant (4) enfermé entre celles-ci, ledit article comportant une partie avant (5), une partie arrière (6) dotée de bords latéraux, et une partie d'entrejambe (7) située entre celles-ci, et il est en outre muni d'une paire de parties ceinture (9) fixées à la partie arrière (6) de l'article et qui sont destinées à être attachées ensemble autour de la taille de l'utilisateur et dans lequel ladite partie avant (5) est munie de moyens d'attache (8) destinés à être fixés aux parties ceinture (9), de telle manière que l'article prenne une forme de culotte dans laquelle les parties ceinture (9) forment une portion des parties taille de l'article absorbant,
**caractérisé en ce que**
les parties ceinture (9), avant l'utilisation, sont pliées en accordéon et forment chacune un paquet (11) plié en accordéon qui est agencé dans une pochette (12) sur chaque bord latéral de la partie arrière (6) de l'article absorbant et qui peut être étendu, à partir de cette pochette et déplié lorsque l'article doit être utilisé, après quoi les parties ceinture peuvent être attachées ensemble autour de la taille de l'utilisateur.

2. Article absorbant selon la revendication 1,
**caractérisé en ce que**
ladite pochette (12) est réalisée entre la feuille supérieure (2) perméable aux liquides et la feuille arrière (3) imperméable aux liquides de la partie arrière (6).

3. Article absorbant selon la revendication 1 ou 2,
**caractérisé en ce que**
les plis (9') en accordéon des parties ceinture (9) peuvent être maintenus ensemble à l'aide de sceaux (13) qui peuvent être rompus.
